# EUROPEAN PATENT SPECIFICATION

(11) **EP 3 972 552 B1**
(45) Date of publication and mention of the grant of the patent: **02.11.2022**
(21) Application number: 20726041.5
(22) Date of filing: 12.05.2020
(51) Int. Cl.: A61K 8/02, A61K 8/06, A61K 8/25, A61K 8/26, A61K 8/73, A61Q 1/00, A61Q 19/08

(54) **COSMETIC COMPOSITIONS FOR SOFT-FOCUS**
KOSMETISCHE ZUSAMMENSETZUNGEN FÜR WEICHZEICHNER
COMPOSITIONS COSMÉTIQUES POUR SOFT FOCUS

(30) Priority: 21.05.2019 WO PCT/CN2019/087738; 02.07.2019 EP 19183831
(43) Date of publication of application: 30.03.2022
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: CAO, Xiujuan, Nanjing 210009 Jiangsu (CN); WANG, Lin, Shanghai, 200335 (CN)
(74) Representative: James, Helen Sarah
(86) International application number: PCT/EP2020/063136
(87) International publication number: WO 2020/234038

(56) References cited:
- EP-A1- 1 072 651
- EP-B1- 1 072 651
- WO-A1-2017/116944
- DE-A1-102011 085 694
- FR-A1- 3 061 004
- US-A- 3 530 217
- US-A1- 2009 324 654
- US-A1- 2011 150 947

## Description

### Field of the invention

The present invention relates to cosmetic compositions. More particularly, the invention relates to cosmetic compositions for blurring or hiding superficial or surface imperfections of skin, such as fine lines and wrinkles by creating optical effects on the skin. Such effects are generally referred to as soft-focus.

### Background of the invention

Our skin is amenable to deterioration due to dermatological disorders, environmental factors such as strong sunlight and due to chrono-ageing, which is accelerated by exposure to sun (photo-ageing). In recent years the demand for cosmetic compositions and cosmetic methods for improving the appearance of skin has grown enormously. Consumers are increasingly seeking cosmetic compositions to treat, delay or conceal the visible signs of chrono-ageing and photo-ageing skin such as wrinkles, fine lines, sagging, hyperpigmentation and age spots.

Imperfections of skin can be hidden in two ways by manipulation of transmission of light. In the first manner, one or more ingredients of the cosmetic composition may simply reflect light. Usually, the scientists who formulate cosmetic compositions rely on talc, silica, kaolin and other inorganic particulate materials which have high refractive indices. An alternative approach is referred to as soft-focus effect, also called blurring effect. In this case, the incident light is distorted by scattering (lensing) and certain ingredients could be used to create such an effect. Such ingredients operate as lenses which bend and twist the light in multiple directions. An example of the above is particulate silicone elastomers.

Soft focus or blurring is a technique which can be used to hide or conceal superficial or surface imperfections of the skin. When particulate ingredients operate as lenses to bend and twist light in many directions, incoming light is distorted by scattering (lensing). In such compositions, the ingredient or ingredients of the cosmetic, operate as lenses to bend and twist light in multiple directions. Wrinkles and fine lines are perceivable primarily as dark, non-reflective areas due to the way light falls and remains there. In contrast if light is reflected and diffused, the wrinkles become less visible. Methods have been developed to project light into the wrinkles and immediately eliminate the dark areas that make the crease obvious. The 'soft focus effect' was described by Dr. Emmert in "Quantification of the Soft-Focus Effect", Cosmetics and Toiletries, Vol. 111, 57-61 (1996).

It is known that certain particulate materials can hide or conceal the superficial imperfections. However, if more of such particles are included in cosmetic compositions, the particles begin to adversely affect the gloss or shine. Therefore, a technical problem is to conceal or hide the surface imperfections, without affecting other attributes including desirable properties of the compositions.

US20190125649 A1 (Unilever) discloses that a combination of porous silica and silicone elastomer boosts or enhances blurring efficacy (soft focus) of the composition.

WO2017220310 A1 (Unilever) discloses a soft-focus composition comprising retinol, porous silica, silicone elastomer. An L&W Index is used to compare efficacy of compositions. This Index is a measure of the performance.

US2007179241A (L'Oreal) discloses a cosmetic composition containing at least one silicone elastomer and at least one encapsulated pigment, wherein refractive index of the pigment is greater than the refractive index of the encapsulating material. It is disclosed that ingredients such as silicone elastomers, planar powders, spherical powders such as silica, PMMA, barium sulfate, etc., and/or powders with a high refractive index such as titanium dioxide, zinc oxide and iron oxide have drawbacks such as, for example, an unnatural look on skin owing to the use of highly opaque materials and the highlighting of skin defects owing to the use of planar materials.

US6432535 BA (Merck) discloses a composite material in which thin flakes of a platelike pigment contain particles of spherical silica having an average particle size of 20 to 400 nm on the surface of the flaky substrate having an average particle size of 0.5 to 10 µm and then the spherical silica is further coated with ultrafine particles of titanium dioxide. The composite particles are good for soft focus effects, spreading, UV protection and whiteness.

US2007179241A (L'Oreal) discloses a soft-focus cosmetic composition containing at least one silicone elastomer and at least one encapsulated pigment whose Refractive Index is greater than that of the encapsulating material (e.g., silica).

US2009324654A (Unilever) discloses a cosmetic composition which includes composite particles of a sunscreen agent and a condensation polymerized polyamide binder, a water-insoluble powdered polymer of porous particles having an Oil Absorbance (castor oil) Value ranging from about 90 to about 500 ml/100 g. The composition exhibits soft-focus which hides skin imperfections and provides sun protection.

WO 2017/071886 A1 (Unilever) discloses cosmetic compositions that comprise turbostratic boron nitride particles and porous silica. This composition is useful for soft-focus without affecting skin lightening.

### Summary of the invention

It has been determined that soft focus benefits provided by a certain class of microspheres can be increased significantly by including a certain kind of a thickener in the cosmetic composition. Therefore, the present inventors believe that there is a synergistic interaction between the ingredients. The combination of ingredients disclosed in this invention provides not only technically superior cosmetic composition but also significantly economical one because formulation scientists can utilise this observation to formulate efficacious cosmetic compositions whilst reducing or lowering the amount of such microspheres. Such an effect is not observed when the microspheres are combined with other conventional thickeners which strengthened the present inventors' belief that synergistic interaction is unexpected. Usually thickeners have a limited role in the context of cosmetic compositions, but we have surprisingly found that certain thickeners can do much more than that, especially when co-formulated with the certain class of microspheres.

In accordance with a first aspect is disclosed a cosmetic composition comprising:
(i) microspheres for providing soft-focus benefits where said microspheres comprise at least 50% cellulose by weight of microspheres; and,
(ii) 0.5 to 10 wt% of a non-silica hydrophilic inorganic thickener; wherein refractive index of said microspheres is from 1.42 to 1.50.

In accordance with a second aspect is disclosed a method of blurring superficial imperfections of skin comprising a step of applying thereon a cosmetic composition of the first aspect.

In accordance with a third aspect is disclosed use of a cosmetic composition of the first aspect for blurring superficial imperfections of skin.

All other aspects of the present invention will more readily become apparent upon considering the detailed description and examples which follow.

### Definitions

### Refractive Index

Refractive index is determined at 25 °C at wavelength of 589 nm.

### Particle Size

Where the size of particles is mentioned (other than "primary particle size") this means the number average diameter determined, for example using scanning electron microscopy (SEM). Similarly, "thickness" means the number average thickness determined, for example using scanning electron microscopy (SEM). In a preferred method, cryo-SEM is used to determine particle size and thickness. In cryo-SEM, a composition is quick-frozen in liquid nitrogen followed by cryo-planing to create a flat surface to be imaged. Typically, the diameter and thickness are each averaged over at least 200 particles. For diameter determination, intact particles are selected from the SEM image whilst for shell thickness, particles which have been sectioned are selected.

If particles are not spherical, e.g., oval or spheroidal, then "diameter" means the largest distance measurable across the particle.

Where primary particle size is mentioned this means the size (diameter) measurable by transmission electron microscopy (TEM) using a method such as that described by S. Gu et al in Journal of Colloid and Interface Science, 289 (2005) pp. 419-426.

"Diameter" as used herein refers to particle diameter in non-aggregated state unless otherwise stated. For polydisperse samples having particulate with diameter no greater than 1 µm, diameter means the z-average diameter measured, for example, using dynamic light scattering (see international standard ISO 13321) with an instrument such as a Zetasizer Nano^{®}. (Malvern Instruments Ltd, UK) unless otherwise stated. For polydisperse samples having particulate with diameter no less than 1 µm means the apparent volume median diameter (D50, also known as .times.50 or sometimes d(0.5)) of the particles measurable for example, by laser diffraction using a system (such as a Mastersizer^{®} 2000 available from Malvern Instruments Ltd) meeting the requirements set out in ISO 13320 unless otherwise stated.

"Skin" as used herein, is meant to include skin on the face, neck, scalp, underarms, chest, back, arms, hands, legs, scalp, feet, buttocks and abdomen, preferably the hands, neck, face, and underarms.

Superficial imperfections of skin mean and include blemishes (age spots, blotches), pores, fine lines and wrinkles, and grooves (the words "lines," "wrinkles," and grooves being used interchangeably herein). "Age spots" as used herein means any hyperpigmentation (e.g. including solar lentigo), spots and/or freckles.

The term treating or treatment as used herein includes within its scope reducing, delaying and/or preventing the abovementioned skin conditions and generally enhancing the quality of skin and improving its appearance and texture.

By "specular reflectance" is meant mirror-like reflection of light from a surface in which light from a single incoming source is reflected into a single outgoing direction. The light may be of any wavelength able to undergo specular reflectance from the surface of facial skin, preferably it is visible light.

By "a cosmetic composition" as used herein, is meant to include a composition for topical application to the skin of humans. Such a composition may be generally classified as leave-on or rinse off but is preferably of the leave-on type. The composition is formulated into a product which is applied to a human body specifically for improving appearance. The composition of the present invention can be in the form of a liquid, lotion, cream, foam, scrub, gel, or toner, or applied with an implement or via a face mask or a pad. Non-limiting examples of such compositions include leave-on skin lotions, creams, foundations, sunless tanners and sunscreen lotions.

Except in the examples, or where otherwise explicitly indicated, all numbers in this description indicating amounts of material or conditions of reaction, physical properties of materials and/or use may optionally be understood as modified by the word "about". All amounts are by weight of the final composition, unless otherwise specified. The term "solid" as used herein means that the material is not fluid at 25°C. It should be noted that in specifying any range of values, a given upper value can be associated with any lower value. For the avoidance of doubt, the word "comprising" is intended to mean "including" but not necessarily "consisting of" or "composed of'. In other words, the listed steps or options need not be exhaustive. The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis*.

### Detailed description

### The composition of the invention

In accordance with a first aspect is disclosed a cosmetic composition comprising:
(i) microspheres for providing soft-focus benefits where said microspheres comprise at least 50% cellulose by weight of microspheres; and,
(ii) 0.5 to 10 wt% of a non-silica hydrophilic inorganic thickener; wherein refractive index of said microspheres is from 1.42 to 1.50.

Imperfect skin can be hidden generally in two ways through manipulation of light transmission. In the first, components of the cosmetic may simply reflect light towards the source. An alternative approach is referred to as achieving a soft-focus effect. Here the incoming light is distorted by scattering (lensing). Components of the color cosmetic in this mechanism operate as lenses to bend and twist light into a variety of directions. Many consumers desire a younger-looking skin devoid of blemishes, fine lines and wrinkles, especially their face. Such a desire is coupled with the fact that consumers want to look radiant and natural in the absence of having an artificial matte look typically provided by traditional foundation-based products which tend to be overly opaque in nature and may have aesthetic and/or cultural negatives.

Attempts at "perfecting" skin have been made. Often, topical compositions with absorbent fillers (e.g., talc, silica, kaolin) are made wherein such inorganic fillers hide skin imperfections by absorbing some light and simply reflecting light back not unlike paint. An alternative approach is referred to as achieving a soft-focus effect. This occurs when incoming light is distorted by scattering (dispersion) wherein light is twisted into a variety of directions. Soft focus is often thought of as a measure similar to haze but applicable to thin product films. Traditional approaches, unfortunately, either hide imperfections in the absence of radiance or result in radiance and healthy glow but with aesthetically displeasing skin appearance, for example, through enhanced visibility of skin topography.

There is an increasing interest to develop composite particles and compositions with composite particles that yield an excellent soft focus.

Microspheres are discrete spherical particles. Depending on their size and composition, microspheres will impart finished products with a variety of effects that include soft-focus. Microspheres can scatter light to obliterate superficial defects of the skin that include fine lines and wrinkles. This effect is called "Soft Focus" or "Optical Blurring." Such microspheres may or may not have any surface treatment or surface coating.

Cosmetic compositions in accordance with this invention comprise microspheres for providing soft-focus benefits where the microspheres comprise at least 50% cellulose by weight of microspheres. It is preferred that the microspheres comprise at least 90 % cellulose by weight of microspheres. The microspheres may preferably comprise a coating of a non-cellulosic material. Optimally the microspheres are entirely made up of cellulose. Preferably the average particle size of said microspheres is 1 to 100 µm. It is further preferred that refractive index of the microspheres is from 1.42 to 1.50. Compositions in accordance with this invention comprise 0.5 to 10 wt% of said microspheres, more preferably 1 to 5 wt% and most preferably 1 to 3.5 wt% of the composition.

It is preferred that cosmetic compositions in accordance with this invention comprise microspheres for providing soft-focus benefits where said microspheres comprise at least 50% cellulose and some examples of commercially available microspheres are CELLULOBEADS^{®} with the variants being D-5, D-10+, D-30, D-50 and USF+. It is generally observed that when the compositions contain any kind of microspheres for providing soft-focus benefits, especially those that comprise at least 50% cellulose, it is necessary to have at least one kind of thickener for stabilizing the microspheres in the composition. The thickener could be of any kind, i.e. inorganic or organic but we have observed that organic thickeners, at least some of them, tend to negatively affect the soft-focus benefits provided by the compositions.

While not strictly necessary, the compositions in accordance with this invention may additionally comprise one or more other type of microspheres, i.e. non-cellulosic microspheres. Preferably such other microspheres are selected from polymer microspheres, mineral microspheres or natural polymer microspheres. When present, it is preferred that the collective amount thereof is from 0.5 to 10 wt%. It is preferred that the mineral microspheres are silica microparticles such as MSS-500/3H, MSS-500/H from Kobo Products Inc or turbostratic boron nitride such as Softouch^{®} Boron Nitride Powder CC6097 from Momentive. It is preferred that the polymer microspheres are Polymethylsilsesquioxane such as DIASPHERE ^{®} KS-500, Methyl Methacrylate crosspolymer such as MSP-930 or nylon microspheres such as TR-1 or SP-10. It is preferred that the natural polymer microspheres are based on Polylactic Acid such as MAKIBEADS ECO^{®}.

Cosmetic compositions of the invention comprise a non-silica hydrophilic inorganic thickener which swells by intercalating a polar liquid. Preferably the compositions comprise 0.5 to 10 wt% of said hydrophilic inorganic thickener. This amount would depend on several other factors such as the wt% of the microspheres and the nature of the cosmetic composition, i.e., a cream or a lotion or a gel. The term non-silica means that the inorganic thickener is not a thickening silica or any other silica-based thickener. More preferably the compositions of this invention comprise 0.5 to 5 wt%, most preferably 0.5 to 3 wt% of the non-silica hydrophilic inorganic. It is preferred that the inorganic thickener is at least one of magnesium-aluminium silicate, a layered silicate, smectite, hectorite, bentonite, montmorillonite or phyllosilicate. More preferably the inorganic thickener is smectite or magnesium-aluminium silicate.

Several grades of magnesium-aluminium silicates are commercially available, for example, the VEEGUM^{®} range of thickeners such as Normal Fast Ultra, VEEGUM^{®} K, VEEGUM^{®} HV, VEEGUM^{®} PURE, VEEGUM^{®} CH, VEEGUM^{®} HS, VEEGUM^{®} D, VEEGUM^{®} Ultra and the VANATURAL range of thickeners such as VANATURAL^{®} MC. The VANATURAL^{®} range is of the bentonite clay type.

While not necessary, the magnesium-aluminium silicate or smectite may be used in the form of combination or blend of the thickener with a natural organic gum or natural organic thickener. An example thereof is VANATURAL^{®} XGB (Bentonite and Xanthan Gum). Other examples include VAN GEL SX (smectite clay and xanthan gum) and VEEGUM CER (smectite clay and cellulose gum). Other such blends could include polyacrylates, carbomers, cellulose, sodium, carboxymethyl cellulose, hydroxyethyl cellulose, hydroxypropyl cellulose, hydroxypropyl methylcellulose, methylcellulose, xanthan gum, sodium carrageenan, sodium alginate, hydroxypropyl guar, gum arabic (acacia) or gum tragacanth.

Other preferred forms of smectite include sodium magnesium silicates, organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite; and mixtures thereof.

Montmorillonites represent clay minerals, which belong to the dioctahedral smectites, and are materials which swell in water but do not become plastic. The layer packets in the 3-layer structure of the montmorillonites can swell as the result of reversible incorporation of water (in a 2-7 fold amount).

As montmorillonites have a large capacity for ion exchange, aluminum can be replaced by Mg, Fe(II), Fe(III), Zn, Pb, Cr, Cu and others. The resulting negative charge of the octahedral layers is balanced by cations, such as Na⁺ (sodium montmorillonite) and Ca²⁺ (calcium montmorillonite) in interlayer positions.

Smectites are characterized by a 2:1 layer structure in which two tetrahedral sheets form on either side of an octahedral sheet through sharing of apical oxygens. As the apical oxygens from the tetrahedral sheet form ditrigonal or hexagonal rings, one oxygen from the octahedral sheet is located on the centre of each ring and is protonated to yield a structural hydroxyl. In 2:1 phyllosilicates, isomorphous substitution of cations having different valencies can lead to charge imbalances within a sheet. These may be partly balanced by the opposite type of charge imbalance in the adjacent sheet (e.g. a positively charged octahedral sheet may offset some of the negative charge associated with a tetrahedral sheet).

The compounds/clay materials belonging to the class of smectites is quite large, and especially the Cs exchanged classes of smectites includes hectorite, saponite, and montmorillonite in addition to vermiculite, and several non-exchangeable phyllosilicates.

Other preferred thickener as per this invention is sodium and calcium montmorillonite, which are part of the smectite group of natural aluminosilicate minerals, and are the most common members. Montmorillonite is the major phase in a type of clays called bentonites. Amongst the calcium and sodium bentonites, sodium bentonite is more preferred.

Montmorillonite is characterized by the substitution of a limited number of octahedral Al³⁺ with Mg^{2+,} which accounts for its negative charge. This is naturally balanced by Na+ between the clay platelets, partially sunk in the hexagonal openings of the silica layer. Because the sodium ions are not structural they can be easily replaced by other positively charged elements or molecules, and are called exchangeable cations. In addition to the charge balancing cations, a tightly held layer of oriented water, about 0.29 nanometers thick, occupies the space between individual flakes. This water requires temperatures well in excess of 100°C for removal. A single VEEGUM^{®} Magnesium Aluminum Silicate or VANATURAL^{®} Bentonite Clay particle is composed of thousands of these sandwiched platelets with exchangeable cations and a layer of water between each.

The trioctahedral analogues of montmorillonite are saponite and hectorite.

The binding effect of inter-platelet water and counterions makes mechanical delamination of smectite clays very difficult but swelling by intercalation with polar liquids and solutions is quite easy. Likewise, in cases where the full surface area of the clay needs to be exposed and/or its rheological properties exploited, hydraulic delamination is relatively simple. When such an inorganic thickener and water are mixed, water penetrates between platelets forcing them further apart. The cations begin to diffuse away from platelet faces. Diffusion (the movement of cations from between platelets out into the water) and osmosis (the movement of water into the space between platelets) then promote delamination until platelets are completely separated.

It is preferred that cosmetic compositions of the invention comprise water. More preferably the compositions comprise 10 to 90wt% water, furthermore preferably 30 to 70wt% water and optimally from 25 to 60 wt% water. The amount of water will depend on the nature of the composition.

It is preferred that the composition is in the form of an emulsion. More preferably the composition is an oil-in-water emulsion. Alternatively, it is a water-in-oil emulsion. Preferred hydrophobic material for use in the oil phase of such emulsions includes emollients such as fats, oils, fatty alcohols, fatty acids, soaps, silicone oils, synthetic esters and/or hydrocarbons.

### Additional Ingredients

It is preferred that the composition additionally comprises one or more of the following ingredients.

"Silicone elastomer" as used herein refers to deformable organopolysiloxane with viscoelastic properties. Preferably the cosmetic composition comprises a silicone elastomer.

It is preferred that the silicone elastomer is cross-linked. The silicone elastomer can be obtained from curable organo-polysiloxanes. Examples in this respect are: addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl terminated diorganopolysiloxane and SiH-containing diorganopolysiloxane; condensation-curing organopolysiloxane compositions which cure in the presence of an organotin compound or a titanate ester, by a condensation reaction between a hydroxyl terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation or electron beams. The silicone elastomer is preferably obtained by addition reaction-curing organopolysiloxane compositions which cure under platinum metal catalysis by the addition reaction between SiH-containing diorganopolysiloxane and organopolysiloxane having silicon-bonded vinyl groups

The silicone elastomer may either be an emulsifying or non-emulsifying cross-linked silicone elastomer or a combination thereof but preferably the silicone elastomer is non-emulsifying. The term "non-emulsifying," as used herein, defines cross-linked silicone elastomer from which poly-oxyalkylene units are absent. The term "emulsifying," as used herein, means cross-linked organo-polysiloxane elastomer having at least one poly-oxyalkylene (e.g., poly-oxyethylene or poly-oxypropylene) unit.

Preferred silicone elastomers are organo-polysiloxanes available under the INCI names of dimethicone/vinyl dimethicone crosspolymer, dimethicone crosspolymer and Polysilicone-11. More preferably the silicone elastomer is dimethicone/vinyl dimethicone crosspolymer.

Preferably, the cosmetic compositions of the invention comprise 0.1 to 10 wt% of the silicone elastomer, more preferably 0.5 to 8 wt%, even more preferably 1 to 5 wt%.

Further preferably the carrier comprises silicones which are not elastomers.

Silicones may be divided into the volatile and nonvolatile variety. Volatile silicone oils (if used) are preferably chosen from cyclic (cyclomethicone) or linear polydimethylsiloxanes containing from 3 to 9, preferably from 4 to 5 silicon atoms.

Compositions of the invention may preferably also comprise a non-silicone emollient. Specific examples of non-silicone emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n- butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, and mixtures thereof.

Of particular use also are the C₁₂₋₁₅ alkyl benzoate esters sold under the Finsolv^{®} brand.

The compositions of the invention may comprise 1 to 25 wt% fatty acid or 0.1 to 80 wt% soap by weight of the composition. Mixtures of fatty acid and soap are also suitable e.g., forming a vanishing cream base which lends a matte feel to the skin. C₁₂₋₂₀ fatty acids are especially preferred, more preferred being C14-18 fatty acids. The most preferred fatty acid is stearic acid, myristic acid or a mixture thereof. When present, the composition comprises 5 to 20 wt% of the fatty acids or soap. Soaps in the hydrophobic material can include alkali metal salt of fatty acids, like sodium or potassium salts, most preferred being potassium stearate. Generally, a vanishing cream base in cosmetic compositions is prepared by taking a desired amount of total fatty matter and mixing with potassium hydroxide in desired amounts. The soap is usually formed in-situ during the mixing.

Preferably, the composition comprises a whitening pigment. The whitening pigment are typically particles of high refractive index materials. For example, the whitening pigment may have a refractive index of greater than 1 .3, more preferably greater than 1 .8 and most preferably from 2.0 to 2.7. Examples of such whitening pigment are those comprising bismuth oxy-chloride barium sulfate, mica, silica, titanium dioxide, zirconium oxide, aluminum oxide, zinc oxide or combinations thereof. More preferred whitening pigment are particles comprising titanium dioxide, zinc oxide, zirconium oxide, mica, iron oxide or a combination thereof. Even more preferred whitening pigment are particles comprising zinc oxide, zirconium oxide, titanium dioxide or a combination thereof as these materials have especially high refractive index. Still even more preferably the whitening pigment is selected from titanium dioxide, zinc oxide or a mixture thereof and most preferred whitening pigment is titanium dioxide.

The average diameter of whitening pigment is typical from 15 nm to 2 µm, more preferably from 35 nm to 800 nm, even more preferably from 50 nm to 500 nm and still even more preferably from 100 to 300 nm. Diameter of whitening pigment refers to the diameter of particles in an un-aggregated state. In the event a well-defined sphere is not generated, diameter means the largest measurable distance on a particle The average diameter may be measured for example by scanning electron microscopy (SEM) or transmission electron microscopy (TEM) by averaging the value of at least one hundred particles.

Preferably the composition comprises 0.001 to 10 wt% whitening pigment, more preferably 0.01 to 6 wt%, more preferably still 0.1 to 3 wt% and most preferably 0.2 to 2 wt% whitening pigment.

Preferably the compositions of the invention comprise one or more organic sunscreens. A wide variety of organic sunscreen is suitable for use in combination with the essential ingredients of this invention. Suitable UV-A / UV-B sunscreen include, 2- hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl-p-dimethyl- amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof. A safe and effective amount of organic sunscreen may be used in the compositions useful in the subject invention. The composition preferably comprises from 0.1 % to 10%, more preferably from 0.1 % to 5%, of organic sunscreen.

The composition of the invention preferably comprises a skin lightening agent. Vitamin B3 compounds (including derivatives of vitamin B3) e.g. niacin, nicotinic acid or niacinamide are the preferred skin lightening agent as per the invention, most preferred being niacinamide. Vitamin B3 compounds, when used, are preferably present in an amount in the range of 0.1 to 10%, more preferably 0.2 to 5% by weight of the composition. Other well-known skin lightening agents may also be included at levels of 0.1 to 10 wt%, more preferably 0.2 to 5 wt. Suitable examples include adapalene, aloe extract, ammonium lactate, anethole derivatives, apple extract, arbutin, azelaic acid, kojic acid, bamboo extract, bearberry extract, bletilla tuber, bupleurum falcatum extract, burnet extract, butyl hydroxy anisole, butyl hydroxy toluene, citrate esters, Chuanxiong, Dang-Gui, deoxyarbutin, 1,3-diphenyl propane derivatives, 2,5-dihydroxybenzoic acid and its derivatives, 2-(4-acetoxyphenyl)-1,3-dithane, 2-(4- hydroxyphenyl)-1,3-dithane, ellagic acid, escinol, estragole derivatives, Fadeout (Pentapharm), Fangfeng, fennel extract, ganoderma extract, gaoben, Gatuline Whitening (Gattlefosse), genistic acid and its derivatives, glabridin and its derivatives, gluco pyranosyl-1-ascorbate, gluconic acid, glycolic acid, green tea extract, 4-hydroxy-5-methyl- 3[2H]-furanone, hydroquinone, 4-hydroxyanisole and its derivatives, 4-hydroxy benzoic acid derivatives, hydroxycaprylic acid, inositol ascorbate, lemon extract, linoleic acid, magnesium ascorbyl phosphate, Melawhite (Pentapharm), morus alba extract, mulberry root extract, 5-octanoyl salicylic acid, parsley extract, phellinus linteus extract, pyrogallol derivatives, 2,4-resorcinol derivatives, 3,5-resorcinol derivatives, rose fruit extract, salicylic acid, Song-Yi extract, 3,4,5-trihydroxybenzyl derivatives, tranexamic acid, vitamins like vitamin B6, vitamin B12, vitamin C, vitamin A, dicarboxylic acids, resorcinol derivatives, extracts from plants viz. Rubia and Symplocos, hydroxycarboxylic acids like lactic acid and their salts e.g. sodium lactate, and mixtures thereof.

Solvents such as ethyl alcohol, isopropanol, acetone, ethylene glycol monoethyl ether, diethylene glycol monobutyl ether, diethylene glycol monoethyl ether and mixtures thereof may also be included at suitable levels.

Humectants include those of the polyhydric alcohol-type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1 ,3-butylene glycol, isoprene glycol, 1 ,2,6-hexanetriol, glycerol, ethoxylated glycerol, propoxylated glycerol and mixtures thereof. The amount of humectant may range, for example, anywhere from 0.5 to 50%, more preferably between 1 and 15% by weight of the composition. Most preferred is glycerol (also known as glycerin). Amounts of glycerin may range, for example, from 0.5% to 50%, more preferably from 1 to 35%, optimally from 2 to 15% by weight of the composition.

It is preferred that the L&W (line and wrinkle) Index of the compositions in accordance with this invention is at least -80%, more preferably -70% to 300%, even more preferably -45% to 200%. The method of measurement of L&W index is described in the Examples.

It is preferred that the cosmetic composition of this invention is a skin care composition. More preferably, the composition is preferably an antiperspirant composition or a face (except eye lids and lips) care composition or a bodycare composition. The skin care composition refers to a composition suitable for topical application to human skin, including leave-on and wash- off products. Preferably the term encompasses a fluid liquid, and particularly a moisturizer rather than a make-up product. Most preferred are leave-on compositions. The term "leave-on" as used with reference to compositions herein means a composition that is applied to or rubbed on the skin and left thereon. The term "wash-off" as used with reference to compositions herein means a skin cleanser that is applied to or rubbed on the skin and rinsed off substantially immediately subsequent to application.

The composition can be formulated in any known format. More preferably it is a cream or a lotion. For underarm applications the compositions of the invention are formulated as a deodorant or antiperspirant, preferably as a roll-on or a cream or stick. The cosmetic compositions of the invention may further comprise other ingredients which are common in the art to enhance physical properties and performances. Suitable ingredients include but are not limited to binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

The compositions may be suitable packed in an appropriately sized packaging or dispenser. Packaging can be a jar or tube as well as any other format typically seen for cosmetic, cream, washing and lotion type products. The compositions may be applied topically and preferably 1 to 4 milligrams of composition is applied per cm² of skin.

### Method and Use

In another aspect the invention provides a method of blurring superficial imperfections of skin comprising a step of applying thereon a cosmetic composition of the first aspect. Preferably the superficial imperfections include fine lines and wrinkles. Further preferably the method is non-therapeutic. In other words, the method of the invention is a cosmetic method that is practised on generally healthy individuals as opposed to therapeutic methods that are practised for alleviation of a therapeutic condition.

In yet another aspect the invention provides use of the composition of the invention for blurring superficial imperfections of skin comprising a step of applying thereon a cosmetic composition of the first aspect. Preferably the superficial imperfections include fine lines and wrinkles. Further preferably the use is non-therapeutic. In other words, the use of the invention is a cosmetic method that is practised on generally healthy individuals as opposed to therapeutic methods that are practised for alleviation of a therapeutic condition.

The following examples are provided to facilitate an understanding of the invention. The examples are not intended to limit the scope of the claims.

### Examples

### Example 1:

A variety of compositions (oil-in-water emulsions) were formulated, which were later subjected to some tests. Some information about the important ingredients used in the compositions is included in Table 1.

**Table 1**

| **Trade name** | **INCl name** | **Diameter/µm** |
|---|---|---|
| MSS-500/3H | Silica (Porous) | 3 |
| DC9509 (63% active content) | Dimethicone/Vinyldimethicone Crosspolymer (and) C12-14 Pareth-12 | 3 |
| Makibeads^{®} 80 | Methyl Methacrylate Crosspolymer | 7 |
| Laponite^{®} XLG | Lithium Magnesium Sodium Silicate | Not applicable (NA) |
| Veegum^{®} | Magnesium Aluminum Silicate | NA |
| Cellulobeads^{®} USF | Cellulose (100% cellulose) | 4 |
| Koboguard^{®} 50AMP | Water (and) Acrylates/Ethylhexyl Acrylate Copolymer (and) Aminomethyl Propanol | NA |
| SimulGel^{®} EG | Sodium Acrylate / Sodium Acryloyldimethyl Taurate Copolymer & Isohexadecane & Polysorbate 80 | NA |
| Aristoflex^{®} HMB | Ammonium Acryloyldimethyltaurate/Beheneth-25 Methacrylate Crosspolymer | NA |

Details of the formulations are shown in Table 2.

**Table 2**

| **Ingredients** | **Sample code and ingredient/wt%** | | | | | | | | | | |
|---|---|---|---|---|---|---|---|---|---|---|---|
| | **A** | **B** | **C** | **D** | **E** | **F** | **G** | **H** | **I** | **J** | **K** |
| SimulGel^{®} EG | - | 2.5 | - | 2.5 | - | 2.5 | - | 2.5 | - | - | 2.5 |
| Laponite^{®} XLG | 2.5 | - | 2.5 | - | - | - | 2.5 | - | 2.5 | 2.5 | - |
| Veegum^{®} | - | - | - | - | 2.5 | - | - | - | - | - | - |
| Cellulobeads^{®} USF | - | - | 3.0 | 3.0 | 3.0 | - | - | - | - | - | - |
| DC9509^{®} | - | - | - | - | - | 4.8 | 4.8 | - | - | - | - |
| MSS^{®}-500/3H | - | - | - | - | - | - | - | 3.0 | 3.0 | - | - |
| Makibeads^{®} 80 | - | - | - | - | - | - | - | - | - | 3.0 | 3.0 |
| Koboguard^{®} 50AMP | 1.0 | | | | | | | | | | |
| Tween^{®} 20 | 2.0 | | | | | | | | | | |
| Propylene Glycol | 2.0 | | | | | | | | | | |
| Cetearyl alcohol | 1.0 | | | | | | | | | | |
| Stearic acid | 1.0 | | | | | | | | | | |
| Aristoflex^{®} HMB | 0.3 | | | | | | | | | | |
| Glyceryl Stearate | 1.0 | | | | | | | | | | |
| Cetiol^{®} Utimate | 5.0 | | | | | | | | | | |
| Crodamol^{®} CAP | 5.0 | | | | | | | | | | |
| Mineral oil | 2.0 | | | | | | | | | | |
| Water and minors to | 100 | | | | | | | | | | |

L&W Index of each of the cosmetic compositions in Table 2 was measured by following an invitro model described hereinafter.

### Calculation of L&W Index

The incident light was reflected and scattered by Bio-skin plates. The specular reflected light kept the same polarization as the incident light whereas the scattering light from the volume (diffused light) was un-polarized. SAMBA^{®} camera acquired successively two images corresponding to two states of polarization (parallel and crossed). The parallel image intensity (P) is contributed from the reflected and the scattered light, and the crossed image intensity (C) is contributed from the scattered light only. The parallel image plus the crossed image is equal to the total image delivered by a traditional camera or perceived by human eye.

Gloss of each pixel was calculated using the formula (P-C)/(P+C). The standard deviation (STD) of gloss is a measure of uniformity of the appearance of skin and the tow are inversely proportional. The L&W (line and wrinkle) Index demonstrates the extent of soft-focus provided by the concerned cosmetic composition and it was calculated by using the following formula.

(STD of gloss degree before applying sample - STD of gloss degree after applying sample)/(STD of gloss degree before applying sample).

The observations are summarised in Table 3.

**Table 3**

| **Example Ref. no.** | **Microspheres/wt%** | **Thickener/wt%** | **L&W Index (average)** | **Standard Deviation** |
|---|---|---|---|---|
| A | NIL | Laponite XLG 2.5% | 12.30% | 3.81% |
| B | NIL | 2.5% SimulGel EG | -120.28% | 5.65% |
| C | 3% Cellulobeads USF | 2.5% Laponite | 47.38% | 8.13% |
| D | 3% Cellulobeads USF | 2.5% Simulgel EG | 19.61% | 7.15% |
| E | 3% Cellulobeads USF+ | 2.5% Veegum | 34.14% | 0.35% |
| F | 4.8% DC9509 | 2.5% Simulgel EG | -56.78% | 1.00% |
| G | 4.8% DC9509 | 2.5% Laponite | 3.74% | 0.89% |
| H | 3% Silica | 2.5% Simulgel EG | 33.83% | 1.64% |
| I | 3% Silica | 2.5% Laponite | 39.60% | 1.32% |
| J | 3% PMMA | 2.5% Laponite | 15.71% | 3.46% |
| K | 3% PMMA | 2.5% Simulgel EG | -10.11% | 9.28% |

Only the compositions C and E are within the invention; rest all are outside the scope.

Comparison of A and B with C, D and E clearly indicates that a composition devoid of any ingredient that provides soft-focus benefits, does not show such an effect.

The L&W Index of C, D and E clearly indicates that inclusion of Cellulobeads makes a significant difference but when Cellulobeads are combined with Simulgel^{®} EG, the L&W Index reduces to an appreciable extent. This indicates that a combination of Cellulobeads with an organic thickener is not a good combination for the L&W Index. However, when the same Cellulobeads are combined with Veegum or Laponite, the L&W Index is very high.

Other examples clearly indicate that when other microspheres that provides soft-focus benefits are combined with the same Laponite or Veegum, the L&W Index remains low.

While silica microbeads pair well with Simulgel EG as well as Laponite, the inventors have observed that the appearance of the film was patchy or unclear which could affect the appearance of skin. appearance benefit. Compositions C, D and E and did not show any patchy or unclear appearance.

## Claims

1. A cosmetic composition comprising:
(i) microspheres for providing soft-focus benefits where said microspheres comprise at least 50% cellulose by weight of microspheres; and,
(ii) 0.5 to 10 wt% of a non-silica hydrophilic inorganic thickener; wherein refractive index of said microspheres is from 1.42 to 1.50.

2. A cosmetic composition as claimed in claim 1 wherein said microspheres comprise at least 90 % cellulose.

3. A cosmetic composition as claimed in claim 1 or 2 wherein average particle size of said microspheres is 1 to 100 µm.

4. A cosmetic composition as claimed in any of claims 1 to 3 wherein said composition comprises 0.5 to 10 wt% of said microspheres.

5. A cosmetic composition as claimed in any of claims 1 to 4 wherein said inorganic thickener is at least one of magnesium-aluminium silicate, a layered silicate, smectite, hectorite, bentonite, montmorillonite or phyllosilicate.

6. A cosmetic composition as claimed in any of claims 1 to 5 wherein said composition comprises water.

7. A cosmetic composition as claimed in claim 6 wherein said composition is in the form of an emulsion.

8. A cosmetic composition as claimed in claim 7 wherein said composition is an oil-in-water emulsion.

9. A method of blurring superficial imperfections of skin comprising a step of applying thereon a cosmetic composition as claimed in any of claims 1 to 8.

10. A method as claimed in claim 9 wherein said superficial imperfections include fine lines and wrinkles.

11. A method as claimed in claim 10 wherein said method is non-therapeutic.

12. Use of a cosmetic composition as claimed in any of claims 1 to 9 for blurring superficial imperfections of skin.

13. Use as claimed in claim 12 wherein said superficial imperfections include fine lines and wrinkles.

## Patentansprüche

1. Kosmetische Zusammensetzung, umfassend:
(i) Mikrokugeln zum Bereitstellen von Soft-Fokus-Vorteilen, wobei die Mikrokugeln mindestens 50 Gewichts-% Cellulose, bezogen auf das Gewicht der Mikrokugeln, umfassen und
(ii) 0,5 bis 10 Gew.-% eines hydrophilen anorganischen Verdickungsmittels, das kein Siliziumdioxid ist,
wobei der Brechungsindex der Mikrokugeln 1,42 bis 1,50 beträgt.

2. Kosmetische Zusammensetzung, wie im Anspruch 1 beansprucht, wobei die Mikrokugeln mindestens 90% Cellulose umfassen.

3. Kosmetische Zusammensetzung, wie im Anspruch 1 oder 2 beansprucht, wobei die durchschnittliche Teilchengröße der Mikrokugeln 1 bis 100 µm beträgt.

4. Kosmetische Zusammensetzung, wie in einem der Ansprüche 1 bis 3 beansprucht, wobei die Zusammensetzung 0,5 bis 10 Gew.-% Mikrokugeln umfasst.

5. Kosmetische Zusammensetzung, wie in einem der Ansprüche 1 bis 4 beansprucht, wobei das anorganische Verdickungsmittel mindestens eines von Magnesium-Aluminium-Silicat, einem Schichtsilicat, Smectit, Hectorit, Bentonit, Montmorillonit oder Phyllosilicat ist.

6. Kosmetische Zusammensetzung, wie in einem der Ansprüche 1 bis 5 beansprucht, wobei die Zusammensetzung Wasser umfasst.

7. Kosmetische Zusammensetzung, wie im Anspruch 6 beansprucht, wobei die Zusammensetzung in Form einer Emulsion vorliegt.

8. Kosmetische Zusammensetzung, wie im Anspruch 7 beansprucht, wobei die Zusammensetzung eine ÖI-in-Wasser-Emulsion ist.

9. Verfahren zum Kaschieren von oberflächlichen Hautunreinheiten, umfassend einen Schritt des Auftragens einer kosmetischen Zusammensetzung, wie in einem der Ansprüche 1 bis 8 beansprucht.

10. Verfahren, wie im Anspruch 9 beansprucht, wobei die oberflächlichen Unreinheiten feine Linien und Falten einschließen.

11. Verfahren, wie im Anspruch 10 beansprucht, wobei das Verfahren nichttherapeutisch ist.

12. Verwendung einer kosmetischen Zusammensetzung, wie in einem der Ansprüche 1 bis 9 beansprucht, zum Kaschieren von oberflächlichen Hautunreinheiten.

13. Verwendung, wie im Anspruch 12 beansprucht, wobei die oberflächlichen Unreinheiten feine Linien und Falten einbeziehen.

## Revendications

1. Composition cosmétique comprenant :
(i) des microsphères pour fournir des bénéfices de flou artistique où lesdites microsphères comprennent au moins 50 % de cellulose en masse de microsphères ; et,
(ii) 0,5 à 10 % en masse d'un épaississant inorganique hydrophile de non-silice ;
dans laquelle l'indice de réfraction desdites microsphères est de 1,42 à 1,50.

2. Composition cosmétique selon la revendication 1, dans laquelle lesdites microsphères comprennent au moins 90 % de cellulose.

3. Composition cosmétique selon la revendication 1 ou 2, dans laquelle la taille moyenne de particule desdites microsphères est de 1 à 100 µm.

4. Composition cosmétique selon l'une quelconque des revendications 1 à 3, dans laquelle ladite composition comprend de 0,5 à 10 % en masse desdites microsphères.

5. Composition cosmétique selon l'une quelconque des revendications 1 à 4, dans laquelle ledit épaississant inorganique est au moins un parmi un silicate de magnésium-aluminium, un silicate en couches, de la smectite, hectorite, bentonite, montmorillonite ou un phyllosilicate.

6. Composition cosmétique selon l'une quelconque des revendications 1 à 5, dans laquelle ladite composition comprend de l'eau.

7. Composition cosmétique selon la revendication 6, dans laquelle ladite composition est dans la forme d'une émulsion.

8. Composition cosmétique selon la revendication 7, dans laquelle ladite composition est une émulsion huile-dans-eau.

9. Procédé de floutage d'imperfections superficielles de la peau comprenant une étape d'application sur son dessus d'une composition cosmétique selon l'une quelconque des revendications 1 à 8.

10. Procédé selon la revendication 9, dans lequel lesdites imperfections superficielles comprennent des rides et ridules.

11. Procédé selon la revendication 10, dans lequel ledit procédé est non-thérapeutique.

12. Utilisation d'une composition cosmétique selon l'une quelconque des revendications 1 à 9 pour flouter des imperfections superficielles de la peau.

13. Utilisation selon la revendication 12, dans laquelle lesdites imperfections superficielles incluent des rides et ridules.
